(19) Europäisches Patentamt
European Patent Office
Office européen des brevets

(11) **EP 1 588 758 B1**

(12) **EUROPEAN PATENT SPECIFICATION**

(45) Date of publication and mention of the grant of the patent:
**07.03.2007 Bulletin 2007/10**

(51) Int Cl.:
**B01F 15/00** (2006.01)

(21) Application number: **05008715.4**

(22) Date of filing: **21.04.2005**

(54) **Agitator**

Rührer

Agitateur

(84) Designated Contracting States:
**AT BE BG CH CY CZ DE DK EE ES FI FR GB GR HU IE IS IT LI LT LU MC NL PL PT RO SE SI SK TR**

(30) Priority: **22.04.2004 EP 04009501**

(43) Date of publication of application:
**26.10.2005 Bulletin 2005/43**

(73) Proprietor: **F.HOFFMANN-LA ROCHE AG**
**4070 Basel (CH)**

(72) Inventors:
• **Eisenkraetzer, Detlef**
**82393 Iffeldorf (DE)**

• **Haug, Andreas**
**82377 Penzberg (DE)**

(74) Representative: **Schreiner, Siegfried et al**
**Roche Diagnostics GmbH,**
**Patent Department (TR-E),**
**P.O. Box 1152**
**82372 Penzberg (DE)**

(56) References cited:
**DE-U- 9 400 938          FR-A- 1 600 744**
**US-A- 4 896 971          US-A- 5 052 892**

## Description

The invention relates to an agitator which is suitable, in particular, for use in bioreactors, and also relates to the use of such an agitator.

In bioreactors, agitators are essentially used to equilibrate temperature differences and differences of concentrations of various constituents. Agitators intensify the heat exchange between the thermostatting elements and the fermentation broth. Furthermore, agitators in bioreactors prevent the cells from sedimenting and thus being inhomogeneously distributed during the fermentation. A further task of agitators in bioreactors is to disperse the gas phase in the fermentation broth.

In biotechnology, various agitator types are used. The most frequently used agitator type is the disc agitator. A standard disc agitator is, for example, the Rushton turbine having six perpendicularly arranged blades. Such an agitator generates a flow radial to the agitator axis. Above and beneath the agitator, flow vortices are formed, which result in a high dispersion effect. A further agitator variant is the inclined-blade agitator. This is an agitator in which the angle of attack of the blades (with respect to the agitator axis) is changeable, but is usually 45°. An inclined-blade agitator comprises mainly an axial transport direction with a radial component and therefore, achieves a highly effective mixing. A disadvantage of disc agitators or inclined-blade agitators is, for example, that such agitators, in particular in the case of high gas load, are easily flooded, and as a result are no longer able to disperse the exiting gas completely. Finally, use is made of propeller agitators in cell fermentation, but to a far lesser extent than standard disc agitators or inclined-blade agitators. The flow in a propeller agitator is preferably directed axially.

Fundamentals on agitator technology in bioreactors are described, for example, in Riet, van't, Tramper, J., Basic Bioreactor Design, Chapter 4: Kinetics, Marcel Dekker Inc., 1991; Tatterson, G.B., Fluid Mixing and Gas Dispersion in Agitated Tanks, McGraw-Hill, Inc., 1991 and Bailey, J.E. and Ollis, D.F., Biochemical Engineering Fundamentals, Second Edition, McGraw-Hill, Inc., 1986. In EP 0 745 666, a bioreactor is described which is equipped with disc agitators. Disc agitators are also described in DE 23 49 106 and DE 23 51 763.

US Patent 4,468,130 describes an inclined-blade agitator having cambered agitator blades, the angle of which changes from 16° over the agitator blade. The angle at the tip of the agitator blade is between 16 and 32°.

US Patent 4,896,971 describes an inclined-blade agitator having twisted agitator blades, the twisting being about 8 to 12°. The angle at the tip of the agitator blade is between 18 and 34°.

US Patent 5,052,892 describes an agitator having agitator blades which are bent once at the centre, the angle between the two parts of the agitator blades being 20 or 30°. US Patent 5,297,938 likewise describes an agitator having bent agitator blades, the angle of which is between 7.5 and 22.5°.

US Patent 5,316,443 describes an agitator having a hook-shaped cross section. US Patent 5,326,226 describes an agitator having twisted agitator blades and an angle of the agitator blades between 25 and 45°.

US Patent 5,791,780 describes an agitator in which the agitator blades have a semicircular or semi-elliptical cross section.

FR 1 600 744 describes an agitator in which the agitator blades have a mainly triangular cross section.

DE 94 00 938 U discloses an agitator in which the agitator blades have a inner main blade connected with an outer side blade.

The invention relates to an agitator having at least two essentially rectangular agitator blades (1) which are arranged radially to the axis of rotation (2) and which are inclined at an angle of attack in the direction of rotation relative to a plane perpendicular to the axis of rotation (2), wherein the agitator blades consist of equally large segments (5) and wherein the angle of attack of the individual agitator blade changing continuously or stepwise from 25° to 35° at the top end to 55° to 65° at the lower end of the agitator blade (preferably from 30° to 60°). The angles of attack of the individual agitator blades (1) are preferably identical. Preferably, the agitator has two to eight, particularly preferably four, agitator blades (1) on one plane. Depending on the size of the bioreactor, agitator blades (1) can be arranged in a plurality of planes above and below one another; one to six planes are expedient.

Preferably, the agitator blades (1) are fixed to a hollow cylinder (3) which serves to receive the agitator shaft (4). If appropriate, the hollow cylinder (3) is joined to the agitator shaft by fixing means. Such a hollow cylinder (3) having agitator blades (1) fixed thereto is termed agitator element hereinafter. The agitator elements therefore preferably have a radially inner hollow ring region which encloses the agitator shaft (4), to which ring region two to eight agitator blades are attached at regular intervals from one another.

Preferably, the agitator blades (1) consist of several segments (5), the angles of attack of which are different.

More preferably, an agitator blade (1) consists of equally large segments (5), the angles of attack of which are different, particularly preferably the angle of attack of the upper segment is between 25° and 35° and of the lower segment between 55° and 65°.

Especially preferably, an agitator blade (1) consists of three equally large segments (5), the angles of attack of which are different, particularly preferably the angle of attack of the upper segment is 30°, of the middle segment 45° and of the lower segment 60°.

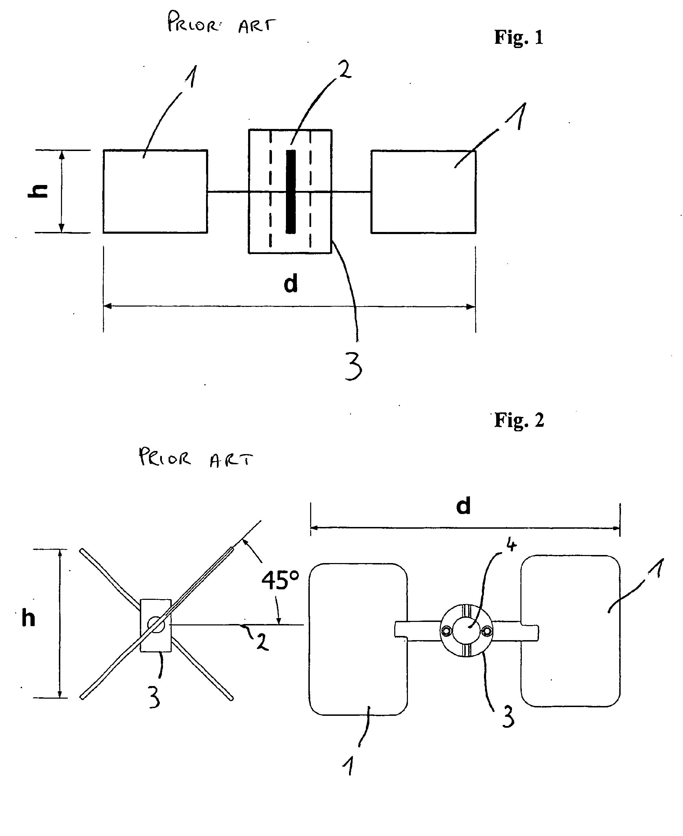

The diameter ratio of a single agitator blade is preferably in the range from 0.3 to 0.5, particularly preferably 0.35 to 0.45 (in accordance with DIN 28131, the ratio d/D, agitator diameter/reactor internal diameter) and the ratio of agitator blade height to agitator diameter (d/h) is preferably 0.1 to 0.3, and particularly preferably 0.1 to 0.15 (see Fig. 1).

Dimensions of preferred agitators are, for example:

| d/D | 0.33 | 0.4 |
|---|---|---|
| h/d | 0.14 | 0.2 |
| d | 350 mm | 350 mm |

The shape of an inventive agitator blade is rectangular, with the edges and corners being able to be rounded. Further preferably, the agitator blade has the shape of a cylindrical section and/or is bent once or several times to achieve the inventive angle of attack.

The shape of the bioreactor is not critical. Usually, a cylindrical vessel is used.

Essential parameters for evaluating efficiency and performance of an agitator are the parameters power input [W/m$^3$], oxygen transport coefficient [$k_L a$ (1/h)], mixing time [S] and cell growth [cell concentration and vitality].

The oxygen transport coefficient $k_L a$ is determined according to the following formula:

$$ k_L a = \frac{OTR}{(C^{\bullet}_{O_2} - C_{O_2})} $$

$k_L a$: oxygen transfer coefficient
OTR: oxygen transfer rate [mol/(l·h)]
$C^{*}_{O2}$: equilibrium concentration of oxygen at the phase boundary
$C_{O2}$: concentration of oxygen in the interior of the suspension [mol/l]

The $k_L a$ value can be determined by various methods. It has been found many times that the measurement range is method-dependent. In the present invention, the "dynamic method" was used to determine $k_L a$ (Zlokarnik, M., Rührtechnik - Theorie und Praxis, Springer Verlag, Heidelberg, New York, 1999).

The examples, references and figures hereinbelow describe the invention, the scope of protection of which is given by the patent claims, in more detail. The methods described are to be taken as examples which, even after modifications, still describe the subject-matter of the invention.

## Description of the Figures

**Figure 1** Diagrammatic drawing agitator

h: Agitator height (projected onto the vertical, see Fig. 2)

**Figure 2** Inclined-blade agitator (SBR Type 1)

**Figure 3** Inclined-blade agitator (SBR Type 2)

**Figure 4** Inventive agitator (SBR Type 3) in paddle form

**Figure 5** Inventive agitator in blade form

**Figure 6** Oxygen transport coefficient as a function of speed of rotation

**Figure 7** Mixing time as a function of speed of rotation

**Figure 8** Graph showing oxygen transport coefficient as a function of speed of rotation for a SBR Type 1 and a SBR Type 3 agitator in a 1000 L fermenter

## Example 1

**Fermenter:**

For the fermentation, use was made of a stirred-tank fermenter having a working volume of 10 l for culturing a CHO cell line. To produce the culture medium the individual constituents are supplemented in heated purified water (type 2) in a sterile make-up vessel. The media osmolality (0.29 Osmol/kg) is set, as in the experimental determination of the $k_L a$ value by NaCl. The pH of 7.1 can be set via addition of correction agents.

The drive unit consists of a bench construction having a suspended electric drive motor. This is a 0.75 kW direct current motor having a range of speed of rotation of 0 to 1500 rpm. To form the supply unit all apparatus, connections and fittings which are required for providing and removing steam, cooling water, wastewater, compressed air, carbon dioxide, nitrogen and correcting agents are combined. These include the heating system, gas mixing station and the pressure control valve. In addition, the electrical energy supply is included in the supply unit.

The bioreactor used has a height-to-diameter ratio (H/D) of 2.0. The reactor is constructed with dished base, flat lid and longitudinal inspection glass. The heating is performed via a jacketed heat exchanger (V = 3L). In the vessel there are four baffles having a width of 0.1 x D.

Three 25 mm and two 19 mm Ingold ports are let into the vessel wall laterally. Depending on requirements of the measurement control instrumentation of the fermentation, pO$_2$ electrode, pH electrode, temperature sensor (PT 100), sampling valve (CV 25), turbidity probe and pCO$_2$ electrode can be used here.

**Agitator elements used:**

The agitator elements consist of stainless steel and are fixed via two grub screws on the agitator shaft which is centrally located in the fermenter. Its direction of rotation was chosen to be anticlockwise. The number of agitator elements per fermenter was three for the standard disc agitator and two for inclined-blade agitators.

**Standard disc agitator (SSR):**

The radially transporting standard disc agitator consists of a horizontally arranged disc to which are symmetrically fixed six perpendicular discs (DIN 28 131). An agitator having a diameter ratio (D/d) of 0.4 was used.

**Inclined-blade agitator type 1 (SBR Type 1) (Fig. 2):**

The angle of attack of the paddles was variable. The oxygen input was studied for 45° (SBR Type 1) and for 60° (SBR Type 1_60°)

Dimensions:
| | |
|---|---|
| d | 118 cm |
| Blade width $d_B$: | 40 cm |
| Blade length: | 95 cm |
| Diameter ratio (d/D = 0.55) | |

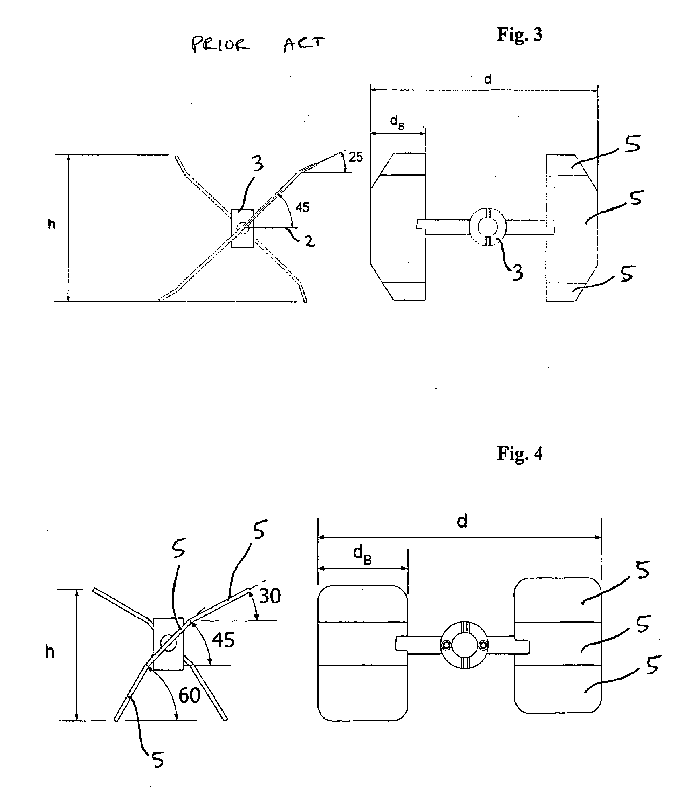

**Inclined-blade agitator type 2 (SBR Type 2) (Fig. 3):**

In contrast to the inclined-blade agitator type 1, the inclined-blade agitator type 2 was designed with a narrower but longer paddle surface. The paddle is slightly bent at the ends. The position of the bent ends is opposed [in Z shape].

Dimensions:
| | |
|---|---|
| d: | 118 cm |
| Blade width $d_B$: | 16/29 cm |
| Blade length: | 115 cm |
| Blade length without bent ends: | 93 cm |
| Diameter ratio (d/D = 0.55) | |

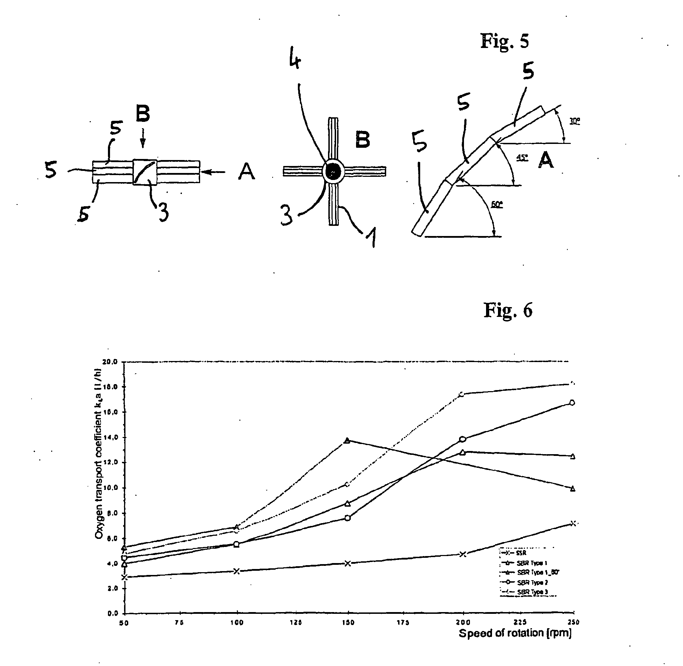

**Inventive inclined-blade agitator (SBR Type 3) (Figs. 4 and 5):**

The inventive inclined-blade agitator SBR Type 3 is subdivided into three equalsized segments (each 32 cm in length). In contrast to the inclined-blade agitator type 2, the outer segments were bent in the same direction [C shape]. The two outer segments were inclined with respect to the central segment in the direction of rotation by 15°.

Dimensions:
| | |
|---|---|
| d/D: | 0.335 |
| d: | 350 mm |
| h/d: | 0.139 |

**Measurement of process parameters:**

**Oxygen measurements:**

The dissolved oxygen concentration was determined using a Clark oxygen electrode (Metler Toledo, InPro® 6000).

**Temperature, pressure and pH measurement:**

The pH and temperature were measured in the fermentation system via probes installed in the probe ring. For the temperature measurement, a PT 100 resistance thermometer was used. Its accuracy is tested during sterilization and at prescribed time intervals during fermentation by a contact thermometer (Type CS 20). The pH was determined via a combination pH electrode from Ingold. Before installation, this electrode was calibrated with buffer solutions pH = 4.01 and pH = 7.0. For pressure measurement, a pressure paste electrode was used.

**Turbidity measurement:**

To determine the mixing times, a turbidity measurement system from Aquasant Messtechnik AG (AS81 with AF44) was used.

**Experimental determination of $k_L a$ value:**

The oxygen transport coefficient was determined by the saturation method.

The fermentation system is charged with 10 of deionized water after installation of the gas-introduction and agitator element under test. All internals (probes, baffles and riser pipes) and process parameters (p = 1 bar, T = 37°C, osmolality = 0.3 Osmol/kg) correspond to those in the fermentation. The osmolality is set and checked by means of NaCl. After heating and calibrating the $pO_2$ electrode, all of the dissolved oxygen can be removed from the medium via gas introduction with nitrogen (Gas1 = 0.51/min, speed of rotation = 250 rpm).

At time point t = 0, pure compressed air introduction starts and the respective speed of rotation is set. The dissolved oxygen concentration increases to the saturation concentration of approximately 6.6 g/l. At the interval of three experiments the $pO_2$ electrode was recalibrated and also in the case of relatively long use of the same medium the osmolality was checked daily.

The $k_L a$ value was calculated via the equation

$$k_L a = - \frac{\ln\left[1 - \left(\frac{c_{O_2, L}}{\overset{\bullet}{c}_{O_2, L}}\right)\right]}{t}$$

It was determined as a function of speed of rotation and the feed gas volumetric flow rate (Gas1). For these parameters, ranges and steps were chosen which correspond to those of the fermentation.

The test liquid used in the experiments was 0.15 molar NaCl solution (8.7 g/l). This has hydrodynamic properties (coalescence behaviour, oxygen saturation concentration) similar to the medium.

By using the inventive inclined-blade agitator type 3, a markedly higher increase in $k_L a$ value could be achieved, compared with all other agitator systems used, not only with increasing speed of rotation but also increasing gas-introduction rate. The fall in $k_L a$ value which occurs at a speed of rotation of 200 rpm when the inclined-blade agitator type 1 was used occurred in this agitator only in the form of a reduced increase. The oxygen transport coefficients achieved by the standard disc agitator at 250 rpm could already be achieved with the inclined-blade agitators at speeds of rotation of 100 to 150 rpm (Fig. 6).

**Determination of mixing time:**

The mixing time was determined using a turbidity measurement system using milk as tracer (all other conditions similar to the $k_L a$ value determination). At a tracer concentration of 5 ml/l, a measurement signal of 85% of the maximum measured value was established. The change in turbidity was followed via the turbidity probe installed in the probe ring and displayed via the compensation recorder. The termination condition for this experiment was a constant measurement signal of 85%. The time to achieve the desired mixing quality of 95% is the mixing time.

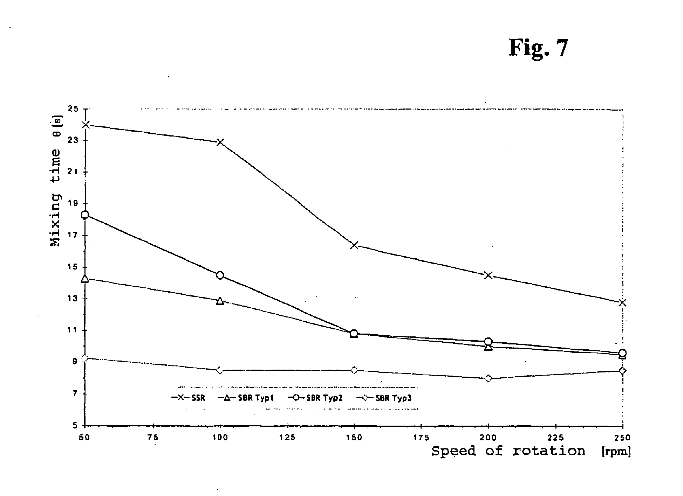

By using the inclined-blade agitator, the mixing times could be decreased by up to 70%. Using the inclined-blade agitator type 3, the mixing time could be decreased by approximately 40% compared with the other inclined-blade agitators (Fig. 7).

**Example 2**

This experiment was performed analogous to the Example 1, but with a stirred-tank fermenter with a working volume of 1000l. The fermenter was filled with a 0.15 mol NaCl water solution at 37 °C.

Two different types of agitators were compared with respect to their oxygen transfer coefficient, $K_L a$, namely a standard inclined-blade agitator (SBR typ 1) and an inventive agitator (SBR typ 3). Before pure compressed air was introduced into the fermenter, oxygen was removed by the introduction of nitrogen (20 L/min, 150 rpm). Afterwards, pure air was introduced at different velocities (Gas 1= 10, 12 or 20 L/min).

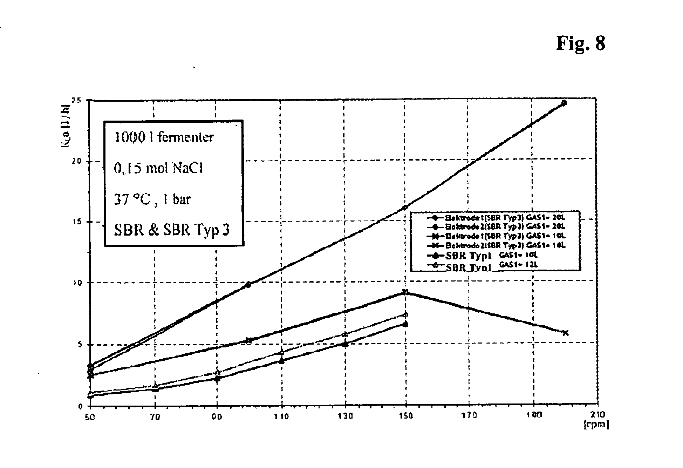

The results of the experiment using two different oxygen electrodes (Elektrode 1 and Elektrode 2) are plotted as "oxygen transfer coefficient"-vs-"speed of rotation" in Figure 8.

By using the inventive inclined-blade agitator type 3, the $k_L a$ value could be increased by a facor of between 1.5 and 2.7 compared with the standard agitator of type 1.

**List of References**

Bailey, J.E. and Ollis, D.F., Biochemical Engineering Fundamentals, Second Edition, McGraw-Hill, Inc., 1986
DE 23 49 106
DE 23 51 763
EP 0 745 666
Riet, van't, Tramper, J., Basic Bioreactor Design, Chapter 4: Kinetics, Marcel Dekker Inc., 1991
Tatterson, G.B., Fluid Mixing and Gas Dispersion in Agitated Tanks, McGraw-Hill, Inc., 1991
US Patent 4,468,130
US Patent 4,896,971
US Patent 5,052,892
US Patent 5,297,938
US Patent 5,316,443
US Patent 5,326,226
US Patent 5,791,780
Zlokarnik, M., Rührtechnik - Theorie und Praxis, Springer Verlag, Heidelberg, New York, 1999

**Claims**

1. Agitator having at least two agitator blades (1) which are arranged radially to the axis of rotation (2) and which are inclined at an angle of attack in the direction of rotation relative to a plane perpendicular to the axis of rotation (2), wherein the agitator blades (1) consist of equally large segments (5) and wherein the angle of attack of the segments of the individual agitator blade (1) changes continuously or stepwise from 25° to 35° at the top end to 55° to 65° at the lower end of the agitator blade.

2. Agitator element according to claim 1, comprising a hollow cylinder (3) to which the agitator blades are fixed and which serves to receive the agitator shaft (4).

3. Agitator according to claim 2, **characterized in that** the hollow cylinder (3) is joined to the agitator shaft (4) by fixing means.

**4.** Agitator according to claims 1 to 3, **characterized in that** the agitator blade (1) consists of three equally large segments (5) and the angles of attack are different.

**5.** Agitator according to claim 4, **characterized in that** the angle of attack of the upper segment is 30°, of the middle segment 45° and of the lower segment 60°.

**6.** Agitator according to Claims 1 to 5, **characterized by** a diameter ratio of 0.3 to 0.5.

**7.** Agitator according to claims 1 to 6, **characterized by** a ratio of agitator height to agitator diameter of 0.1 to 0.3.

**8.** Agitator according to claims 1 to 7, **characterized by** two to eight agitator blades (1).

## Patentansprüche

**1.** Rührer, welcher wenigstens zwei Rührblätter (1) hat, welche radial zu der Rotationsachse (2) angeordnet sind und welche mit einem Anstellwinkel in Rotationsrichtung relativ zu einer Ebene senkrecht zu der Rotationsachse (2) geneigt sind, wobei die Rührblätter (1) aus gleich großen Segmenten (5) bestehen und wobei der Anstellwinkel der Segmente des individuellen Rührblatts (1) sich kontinuierlich oder schrittweise von 25° bis 35° am oberen Ende bis 55° bis 65° am unteren Ende des Rührblatts ändert.

**2.** Rührerelement gemäß Anspruch 1, umfassend einen hohlen Zylinder (3), an welchem die Rührblätter befestigt sind und welcher dazu dient, die Rührerachse (4) aufzunehmen.

**3.** Rührer gemäß Anspruch 2, **dadurch gekennzeichnet, dass** der hohle Zylinder (3) mit der Rührerachse (4) durch Befestigungsmittel verbunden ist.

**4.** Rührer gemäß Ansprüchen 1 bis 3, **dadurch gekennzeichnet, dass** das Rührblatt (1) aus drei gleich großen Segmenten (5) besteht und dass die Anstellwinkel verschieden sind.

**5.** Rührer gemäß Anspruch 4, **dadurch gekennzeichnet, dass** der Anstellwinkel des oberen Segments 30° beträgt, des mittleren Segments 45° und des unteren Segments 60°.

**6.** Rührer gemäß Ansprüchen 1 bis 5, **gekennzeichnet durch** ein Durchmesserverhältnis von 0,3 bis 0,5.

**7.** Rührer gemäß Ansprüchen 1 bis 6, **gekennzeich-**

**net durch** ein Verhältnis der Rührerhöhe zum Rührerdurchmesser von 0,1 bis 0,3.

**8.** Rührer gemäß Ansprüchen 1 bis 7, **gekennzeichnet durch** zwei bis acht Rührblätter (1).

## Revendications

**1.** Agitateur comportant au moins deux pales d'agitateur (1) qui sont agencées radialement à l'axe de rotation (2) et qui sont inclinées suivant un angle d'attaque dans le sens de rotation par rapport à un plan perpendiculaire à l'axe de rotation (2), dans lequel les pales d'agitateur (2) sont constituées de segments de même grandeur (5) et dans lequel l'angle d'attaque des segments de la pale d'agitateur individuelle (1) varie continuellement ou par pas de 25° à 35° à l'extrémité supérieure et à 55° à 65° à l'extrémité inférieure de la pale d'agitateur.

**2.** Elément d'agitateur selon la revendication 1, comprenant un cylindre creux (3) auquel les pales d'agitateur sont fixées et qui sert à recevoir l'arbre d'agitateur (4).

**3.** Agitateur selon la revendication 2, **caractérisé en ce que** le cylindre creux (3) est relié à l'arbre d'agitateur (4) par un moyen de fixation.

**4.** Agitateur selon les revendications 1 à 3, **caractérisé en ce que** la pale d'agitateur (1) est constituée de trois segments de grandeur égale (5) et les angles d'attaque sont différents.

**5.** Agitateur selon la revendication 4, **caractérisé en ce que** l'angle d'attaque du segment supérieur est de 30°, celui du segment intermédiaire est de 45° et celui du segment inférieur est de 60°.

**6.** Agitateur selon les revendications 1 à 5, **caractérisé par** un rapport diamétral de 0,3 à 0,5.

**7.** Agitateur selon les revendications 1 à 6, **caractérisé par** un rapport de hauteur d'agitateur sur diamètre d'agitateur de 0,1 à 0.3.

**8.** Agitateur selon les revendications 1 à 7, **caractérisé par** deux à huit pales d'agitateur (1).

PRIOR ART

**Fig. 1**

PRIOR ART

**Fig. 2**

45°

PRIOR ACT

**Fig. 3**

**Fig. 4**

Fig. 5

Fig. 6

**Fig. 7**

**Fig. 8**